# FASCICULE DE BREVET EUROPEEN

(11) **EP 3 541 759 B1**
(45) Date de publication et mention de la délivrance du brevet: **07.06.2023**
(21) Numéro de dépôt: 17808096.6
(22) Date de dépôt: 07.11.2017
(51) Int. Cl.: C03B 27/06, A61M 5/30

(54) **PROCÉDÉ ET APPAREIL DE TREMPE À L'AIR D'UN CORPS CREUX EN VERRE ALLONGÉ COMPRENANT UN PERÇAGE AXIAL, PRODUIT ASSOCIÉ**
VERFAHREN UND VORRICHTUNG ZUR LUFTABSCHRECKUNG EINES LÄNGLICHEN HOHLEN GLASKÖRPERS MIT AXIALER BOHRUNG, DAZU GEHÖRENDEM PRODUCKT
METHOD AND APPARATUS FOR AIR-QUENCHING AN ELONGATED HOLLOW GLASS BODY COMPRISING AN AXIAL BORE, ASSOCIATED PRODUCT

(30) Priorité: 15.11.2016 FR 1661064
(43) Date de publication de la demande: 25.09.2019
(73) Titulaire: Crossject, 21000 Dijon (FR)
(72) Inventeur: AURIEL, Christophe, 21270 Binges (FR); VIGOT, Xavier, 21260 Veronnes (FR)
(74) Mandataire: Germain Maureau
(86) Numéro de dépôt international: PCT/FR2017/053036
(87) Numéro de publication internationale: WO 2018/091798

(56) Documents cités:
- FR-A1- 2 815 544
- US-A- 3 173 273
- US-A- 3 208 229
- US-A1- 2003 000 227
- US-A1- 2006 016 220

## Description

La présente invention concerne un procédé de trempe à l'air d'un corps creux en verre, ainsi qu'une installation de trempe mettant en oeuvre un tel procédé de trempe, et un dispositif d'injection sous la peau contenant un tel corps creux.

Un type de dispositif d'injection connu, présenté notamment par le document FR-A1-2815544, est prévu pour réaliser sans aiguille des injections intradermiques, sous-cutanées ou intramusculaires, de principes actifs contenus dans un fluide à usage thérapeutique en médecine pour l'homme ou en médecine vétérinaire. Le fluide peut être un gel ou un liquide plus ou moins visqueux.

Ces dispositifs à usage unique contiennent une source d'énergie comme un générateur de gaz sous pression, délivrant un gaz brutalement libéré sur un piston ajusté dans un cylindre formé par un tube en verre, pour propulser le fluide contenu en dessous de ce piston vers une buse d'injection en contact avec la peau, et l'injecter en dessous de cette peau.

Le tube en verre doit présenter des dimensions précises afin de réaliser les étanchéités aux extrémités, et autour du piston coulissant dedans. De plus ce tube doit présenter une résistance mécanique élevée pour résister au choc venant de la pression brutale établie à l'intérieur par le gaz sous pression.

Pour obtenir une résistance mécanique importante d'un tube en verre présentant un axe principal, un procédé de trempe à l'air connu, présenté notamment par le document US-A1-20060016220, utilise une installation de trempe réalisant un refroidissement par projection d'air, comportant huit colonnes réparties autour du tube parallèlement à ce tube, comprenant chacune une série de buses discrètes s'étendant sur la hauteur de ce tube, et dirigées radialement vers l'axe, et une buse axiale venant au dessus du perçage du tube.

Après avoir chauffé le tube à la température requise, l'ensemble des buses est brutalement alimenté en air sous pression pour refroidir en même temps l'extérieur par les buses radiales, et l'intérieur par le jet axial venant au-dessus du perçage du tube.

On obtient un refroidissement brutal formant une trempe des couches superficielles des deux surfaces de la paroi du tube, à l'intérieur et à l'extérieur de ce tube, et un refroidissement plus lent de la matière se trouvant entre les deux couches superficielles de cette paroi. On a alors une précontrainte de pression sur les couches superficielles, et une tension de précontrainte dans la matière à l'intérieur entre ces couches, qui donne une résistance élevée au tube ainsi traité.

Toutefois ce procédé pose des problèmes de répartition inégale de la vitesse de refroidissement sur les surfaces intérieures de la paroi du tube, qui donne à ce tube une résistance variable suivant la hauteur du perçage.

La présente invention a notamment pour but d'éviter ces inconvénients de la technique antérieure.

Elle propose à cet effet un procédé de trempe à l'air d'un corps creux en verre allongé suivant un axe principal, comportant une paroi présentant une surface extérieure, et une surface intérieure formée par un perçage s'étendant en hauteur suivant l'axe principal, ce procédé utilisant des buses de projection d'air dirigées vers les surfaces, étant remarquable en ce qu'il projette simultanément des jets d'air par des buses extérieures réparties sur la surface extérieure, et au-dessus du perçage dans l'axe un jet d'air intérieur formant dans un plan transversal une couronne présentant au centre un creux.

Un avantage de ce procédé de trempe est qu'en plus des jets d'air sur l'ensemble de la surface extérieure, la projection à l'intérieur du perçage du jet d'air creux disposé suivant l'axe, dirige directement l'air de ce jet sur les parois intérieures pour les refroidir au plus vite, sans perte inutile de débit d'air au centre qui traverserait le perçage en ressortant de l'autre côté sans contribuer à refroidir les parois.

On obtient une meilleure évacuation de l'air chaud, et avec un même débit une efficacité maximale sur la paroi interne du tube, donnant un refroidissement plus uniforme sur toute la hauteur.

Le procédé de trempe selon l'invention peut comporter de plus une ou plusieurs des caractéristiques suivantes, qui peuvent être combinées entre elles.

Avantageusement, le procédé de trempe projette de l'air par une buse axiale débouchant au-dessus du perçage.

Dans ce cas, avantageusement le procédé projette de l'air par une buse axiale comprenant une forme qui débouche formant une couronne.

Avantageusement, le procédé projette de l'air par des buses extérieures présentant une fente axiale. On peut obtenir aussi une meilleure répartition de l'air frais sur les surfaces extérieure et intérieure.

Avantageusement, toutes les buses extérieures présentent une fente axiale qui s'étend sensiblement sur toute la hauteur de la surface extérieure à traiter.

Avantageusement, pendant la trempe le procédé met en rotation autour de l'axe principal le jet d'air par rapport au corps creux. On régularise le refroidissement sur le contour des surfaces intérieure et extérieure.

L'invention a aussi pour objet une installation de trempe d'un corps creux en verre telle que définie dans la revendication 7, comportant des buses de projection d'air sur ce corps, remarquable en ce qu'il comporte des moyens mettant en oeuvre un procédé comprenant l'une quelconque des caractéristiques précédentes.

Selon l'invention, l'installation comporte une buse axiale présentant une forme qui débouche comprenant un noyau axial relié par des rayons à un contour extérieur.

L'invention a de plus pour objet un dispositif d'injection réalisant sans aiguille des injections intradermiques, sous-cutanées ou intramusculaires de principes actifs contenus dans un fluide à usage thérapeutique, remarquable en ce qu'il comporte un réservoir contenant ce fluide, formé par un tube en verre constituant un corps creux réalisé par un procédé comprenant l'une quelconque des caractéristiques précédentes.

En particulier, le réservoir peut contenir un fluide présentant au moins un principe actif choisi parmi le groupe comprenant les principes actifs de traitement suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

L'invention sera mieux comprise et d'autres caractéristiques et avantages apparaîtront plus clairement à la lecture de la description ci-après donnée à titre d'exemple, en référence aux dessins annexés dans lesquels :
- la figure 1 est une vue en coupe axiale d'une installation de trempe d'un tube en verre, mettant en oeuvre un procédé de trempe selon l'invention ;
- la figure 2 est une vue en coupe transversale suivant le plan de coupe II-II, d'un cylindre extérieur de projection d'air de cette installation ;
- la figure 3 est un graphique présentant des mesures de contraintes résiduelles dans la paroi de ce tube en verre, avec un procédé de trempe selon l'art antérieur ;
- la figure 4 est un graphique similaire pour un procédé de trempe suivant l'invention ;
- la figure 5 est un graphique présentant des courbes de refroidissement de ce tube en verre pour les deux procédés de trempe présentés ci-dessus ; et
- les figures 6 et 7 présentent en vue de face et en coupe transversale suivant le plan de coupe VII-VII, la buse axiale de cette installation de trempe.

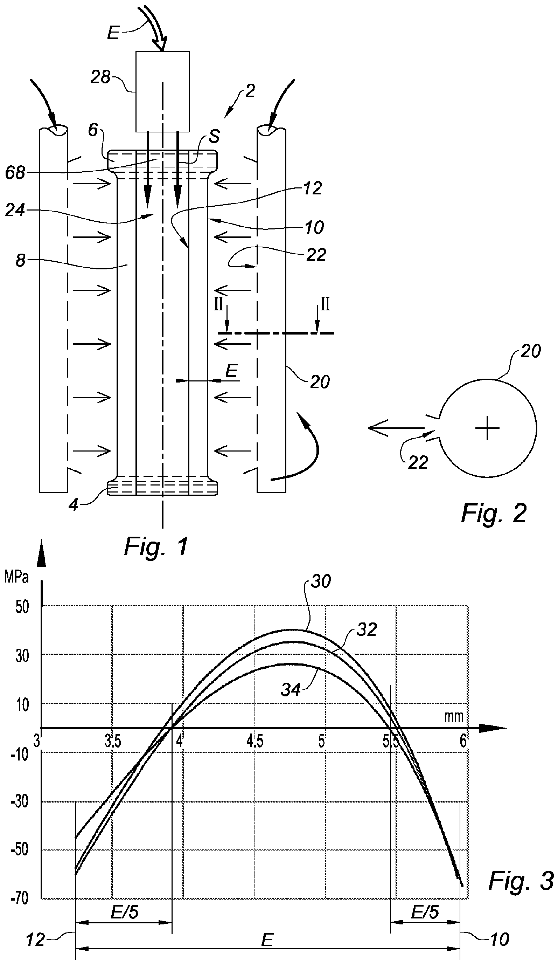

La figure 1 présente un tube en verre 2 de révolution autour d'un axe présenté verticalement, comportant une partie cylindrique tubulaire 8 comprenant une paroi d'épaisseur constante E, se terminant en bas par un bourrelet inférieur 4, et en haut par un bourrelet supérieur 6 présentant une hauteur un peu plus importante.

Sur la figure 1 l'axe est présenté verticalement, la partie supérieure étant nommée le haut par convention, le tube 2 pouvant présenter toutes les orientations pendant le procédé de trempe.

Le tube 2 présente un perçage axial comprenant une section circulaire constante sur toute la hauteur, laissant sur la partie cylindrique 8 une paroi comportant une épaisseur E relativement importante de plusieurs millimètres, qui est sensiblement égale au rayon du perçage.

Les bourrelets inférieur 4 et supérieur 6 donnent une rigidité importante aux extrémités du tube 2, et forment des surfaces d'extrémité transversales planes recevant une étanchéité. Le tube 2 présente une hauteur totale d'environ 30mm.

Le tube en verre 2 fait partie d'un dispositif d'injection présenté notamment par le document de l'art antérieur cité ci-dessus, recevant dans son perçage axial un piston soumis du côté supérieur à une décharge brutale d'un gaz sous pression, afin d'injecter sous la peau un fluide se trouvant dans la partie inférieure.

Le choc brutal de la pression de gaz met en pression le fluide. La paroi de la partie cylindrique 8 du tube 2 doit résister au choc de pression venant du gaz et transmis au fluide.

La figure 3 représente pour un procédé de trempe présenté notamment par le document de l'art antérieur cité ci-dessus, en fonction de la position dans l'épaisseur de la paroi de la partie cylindrique 8 du tube 2, représentée par le rayon par rapport à l'axe de ce tube exprimé en millimètre, une mesure de la contrainte résiduelle dans cette paroi effectuée par photoélasticimétrie à différentes hauteurs de cette partie cylindrique.

La contrainte résiduelle exprimée en MPa, comporte une première courbe 30 de mesures effectuées sur les 5mm supérieurs de la partie cylindrique 8 du tube 2, une deuxième courbe 32 de mesures effectuées sur les 5mm médians, et une troisième courbe 34 de mesures effectuées sur les 5mm inférieurs de cette partie cylindrique.

La photoélasticimétrie permet de visualiser les contraintes existantes dans la matière à l'intérieur des parois grâce à leur photoélasticité, en utilisant la réfringence d'un rayonnement optique traversant cette matière soumise à des contraintes. On étudie la polarisation de la lumière transformée après le passage au travers du matériau.

On constate pour les trois courbes 30, 32, 34 mesurées sur la partie cylindrique 8 à trois hauteurs différentes, l'application de contraintes de compression correspondant à des contraintes négatives, sur des épaisseurs en partant des surfaces intérieure 12 et extérieure 10, qui sont de l'ordre de 1/5 de l'épaisseur E de la paroi du tube.

Toutefois on constate que les contraintes négatives appliquées sur la paroi interne du tube présentent une forte inégalité suivant la hauteur dans le tube 2, qui donne une résistance de ce tube fortement variable.

Les figures 1 et 2 présentent autour du tube 2 une série de cylindres extérieurs 20 disposés parallèlement à ce tube, répartis régulièrement autour de ce tube, présentant chacun une buse extérieure 22 tournée vers l'axe, formant une fente continue disposée en face de la hauteur à traiter de ce tube.

Une buse axiale 28 disposée dans l'axe au-dessus du perçage du tube 2, délivre dans ce perçage un débit d'air formant dans un plan transversal une couronne, présentant au centre un creux 68 où ce débit est absent.

Les cylindres extérieurs 20 ainsi que la buse axiale 28 sont alimentés à l'extrémité supérieure par un débit d'air frais important.

L'installation comporte une motorisation des cylindres extérieurs 20, qui entraîne ces cylindres et donc les jets d'air projetés en rotation rapide suivant l'axe au cours de la trempe à l'air.

De cette manière on obtient à la fois une bonne répartition axiale du flux d'air à l'extérieur grâce aux fentes continues des buses extérieures 22, ainsi qu'une bonne répartition angulaire grâce à la rotation des cylindres 20 permettant de présenter les flux de manière équivalente sur l'ensemble du contour de la surface extérieure 10.

On obtient aussi une optimisation du débit d'air à l'intérieur du perçage, le jet d'air avec le creux intérieur 68 formant une couronne qui concentre cet air sur la surface intérieure 12. Avec un débit d'air équivalent, on obtient un meilleur refroidissement de cette surface intérieure 12 sur l'ensemble de la hauteur.

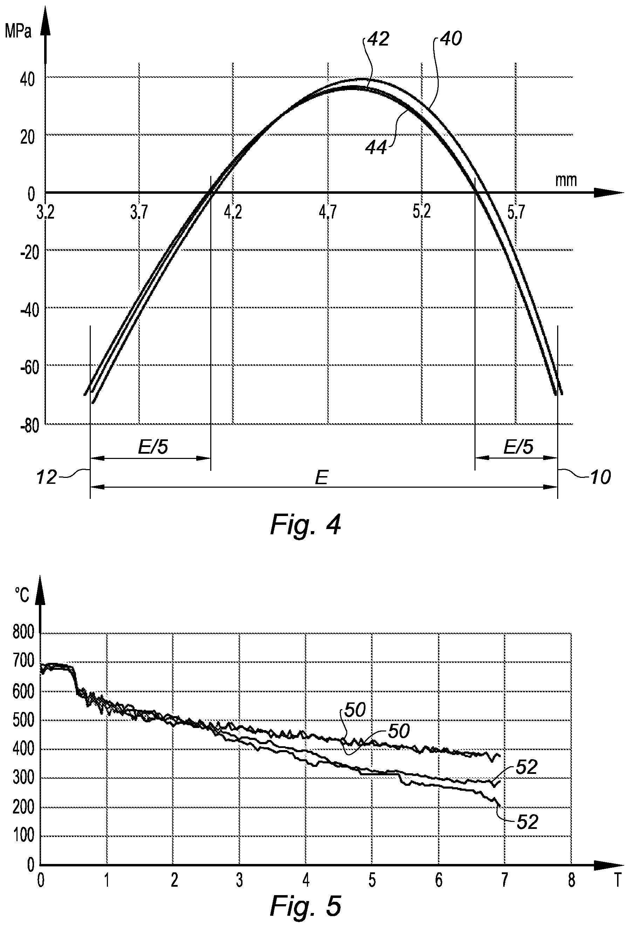

La figure 4 présente pour le procédé de trempe selon l'invention, en fonction de l'épaisseur E de la paroi de la partie cylindrique 8 du tube 2, la contrainte résiduelle à l'intérieur de cette paroi.

On a une première courbe 40 de mesures effectuées sur les 5mm supérieurs de la partie cylindrique 8 du tube 2, une deuxième courbe 42 de mesures effectuées sur les 5mm du plan transversal médian, et une troisième courbe 44 de mesures effectuées sur les 5mm inférieurs de cette partie cylindrique.

On constate aussi pour les trois courbes 40, 42, 44 mesurées sur la partie cylindrique 8 à trois hauteurs différentes, l'application de contraintes de compression correspondant à des contraintes négatives, sur des épaisseurs en partant des surfaces intérieure 12 et extérieure 10, qui sont de l'ordre de 1/5 de l'épaisseur E de la paroi du tube.

Toutefois on constate que les contraintes négatives appliquées sur la paroi interne du tube présentent une bonne égalité suivant la hauteur dans le tube 2, qui donne une résistance de ce tube très constante.

On obtient donc une grande homogénéité de la résistance de l'ensemble du tube 2, qui est proche de la résistance maximale obtenue avec le procédé selon l'art antérieur.

La figure 5 présente en fonction du temps T exprimé en seconde sur l'axe horizontal, la température maximale mesurée sur le tube au cours de la phase de refroidissement, exprimée en °C.

Deux premières courbes 50 représentent la température maximale pour un procédé selon l'art antérieur présenté ci-dessus, et deux deuxièmes courbes 52 représentent la température pour un procédé selon l'invention.

On constate pour l'ensemble des courbes 50, 52 une descente équivalente de la température jusqu'au temps égal à 2,5s, avec une température atteinte d'environ 480°C. On constate ensuite une descente plus rapide de la température pour les deux deuxièmes courbes 52, qui atteint au bout de 6s une température un peu inférieure à 300°C, alors que la température des premières courbes 50 est encore à 400°C.

On obtient avec le procédé suivant l'invention à la fois une baisse de température plus rapide, ainsi qu'une meilleure répartition de cette baisse de température qui donne les meilleures qualités de résistance au tube 2.

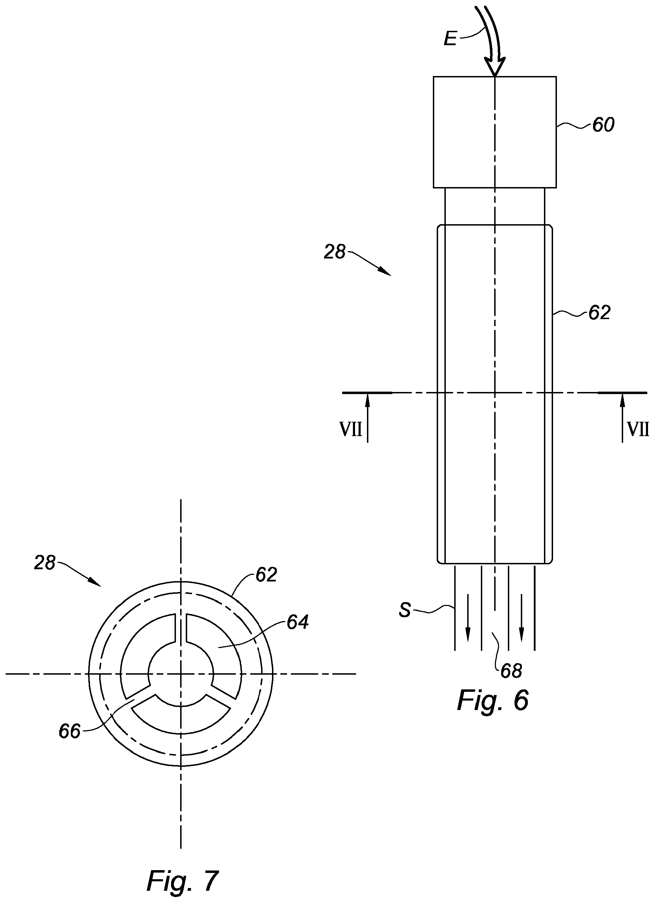

Les figures 6 et 7 présentent une buse axiale 28 comprenant une entrée E et une sortie S, comportant en partie inférieure un filetage extérieur 62 prévu pour se visser un support, la tête supérieure 60 formant une empreinte prévue pour assurer avec une clé un serrage sur ce support.

La partie inférieure de la buse axiale 28 comporte une forme débouchant vers le bas, comprenant un noyau axial relié par trois rayons 66 de faible épaisseur au contour extérieur. De cette manière on dispose de trois passages d'air formant des arcs de cercle 64, qui délivrent le jet d'air constituant une couronne comprenant le creux central 68.

On peut réaliser avec le procédé selon l'invention une trempe à l'air sur un corps creux allongé présentant différentes formes, comprenant un perçage axial.

Le procédé est particulièrement adapté pour un tube d'un dispositif d'injection sous la peau, formant un réservoir contenant un fluide comportant au moins un principe actif choisi parmi le groupe comprenant les principes actifs de traitement suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Revendications

1. - Procédé de trempe à l'air d'un corps creux en verre (2) allongé suivant un axe principal, comportant une paroi présentant une surface extérieure (10), et une surface intérieure (12) formée par un perçage s'étendant en hauteur suivant l'axe principal, ce procédé utilisant des buses de projection d'air (22, 28) dirigées vers les surfaces, **caractérisé en ce qu'**il projette simultanément des jets d'air par des buses extérieures (22) réparties sur la surface extérieure (10), et au-dessus du perçage dans l'axe par un jet d'air intérieur formant dans un plan transversal une couronne présentant au centre un creux (68).

2. - Procédé de trempe selon la revendication 1, **caractérisé en ce qu'**il projette de l'air par une buse axiale (28) débouchant au-dessus du perçage.

3. - Procédé de trempe selon la revendication 2, **caractérisé en ce qu'**il projette de l'air par une buse axiale (28) comprenant une forme qui débouche formant une couronne (64).

4. - Procédé de trempe selon l'une quelconque des revendications précédentes, **caractérisé en ce qu'**il projette de l'air par des buses extérieures (22) présentant une fente axiale.

5. - Procédé de trempe selon la revendication 4, **caractérisé en ce que** toutes les buses extérieures (22) présentent une fente axiale qui s'étend sensiblement sur toute la hauteur de la surface extérieure (10) à traiter.

6. - Procédé de trempe selon l'une quelconque des revendications précédentes, **caractérisé en ce que** pendant la trempe il met en rotation autour de l'axe principal le jet d'air par rapport au corps creux (2).

7. - Installation de trempe d'un corps creux en verre (2), comportant des buses de projection d'air (22, 28) sur ce corps, **caractérisée en ce qu'**elle comporte une buse axiale (28) présentant une forme qui débouche comprenant un noyau axial relié par des rayons (66) à un contour extérieur et des moyens mettant en oeuvre un procédé selon l'une quelconque des revendications précédentes.

8. - Dispositif d'injection réalisant sans aiguille des injections intradermiques, sous-cutanées ou intramusculaires de principes actifs contenus dans un fluide à usage thérapeutique, **caractérisé en ce qu'**il comporte un réservoir contenant ce fluide, formé par un tube en verre constituant un corps creux (2) réalisé par un procédé selon l'une quelconque des revendications 1 à 6.

9. - Dispositif d'injection selon la revendication 8, **caractérisé en ce que** le réservoir contient un fluide présentant au moins un principe actif choisi parmi le groupe comprenant les principes actifs de traitement suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Verfahren zum Abschrecken mit Luft eines entlang einer Hauptachse langgestreckten Hohlkörpers aus Glas (2), der eine Wand beinhaltet, die eine äußere Oberfläche (10) und eine innere Oberfläche (12) aufweist, die durch eine Bohrung gebildet wird, die sich in der Höhe entlang der Hauptachse erstreckt, wobei dieses Verfahren Luftstrahldüsen (22, 28) verwendet, die zu den Oberflächen gerichtet sind, **dadurch gekennzeichnet, dass** es gleichzeitig Luftstrahlen durch äußere Düsen (22), die auf der äußeren Oberfläche (10) verteilt sind strahlt, und oberhalb der Bohrung in der Achse, durch einen inneren Luftstrahl, der in einer Querebene einen Kranz bildet, der in der Mitte eine Vertiefung (68) aufweist.

2. Verfahren zum Abschrecken nach Anspruch 1, **dadurch gekennzeichnet, dass** es Luft durch eine axiale Düse (28) strahlt, die oberhalb der Bohrung einmündet.

3. Verfahren zum Abschrecken nach Anspruch 2, **dadurch gekennzeichnet, dass** es Luft durch eine axiale Düse (28) strahlt, die eine Form umfasst, die durch Bilden eines Kranzes (64) einmündet.

4. Verfahren zum Abschrecken nach einem der vorstehenden Ansprüche, **dadurch gekennzeichnet, dass** es Luft durch äußere Düsen (22) strahlt, die einen axialen Schlitz aufweisen.

5. Verfahren zum Abschrecken nach Anspruch 4, **dadurch gekennzeichnet, dass** alle äußeren Düsen (22) einen axialen Schlitz aufweisen, der sich im Wesentlichen über die gesamte Höhe der zu behandelnden äußeren Oberfläche (10) erstreckt.

6. Verfahren zum Abschrecken nach einem der vorstehenden Ansprüche, **dadurch gekennzeichnet, dass** es den Luftstrahl während des Abschreckens in Bezug auf den Hohlkörper (2) um die Hauptachse herum in Drehung versetzt.

7. Installation zum Abschrecken eines Hohlkörpers aus Glas (2), die Luftstrahldüsen (22, 28) auf diesem Körper beinhaltet, **dadurch gekennzeichnet, dass** sie eine axiale Düse (28) beinhaltet, die eine Form aufweist, die einen axialen Kern umfassend einmündet, der über Speichen (66) mit einer äußeren Kontur verbunden ist, und Mittel, die ein Verfahren nach einem der vorstehenden Ansprüche umsetzen.

8. Injektionsvorrichtung, die ohne Nadel intradermale, subkutane oder intramuskuläre Injektionen von Wirkstoffen durchführt, die in einer Flüssigkeit zur therapeutischen Verwendung enthalten sind, **dadurch gekennzeichnet, dass** sie einen Tank beinhaltet, der diese Flüssigkeit enthält, der durch eine Glasröhre gebildet wird, die einen Hohlkörper (2) darstellt, der durch ein Verfahren nach einem der Ansprüche 1 bis 6 realisiert wird.

9. Injektionsvorrichtung nach Anspruch 8, **dadurch gekennzeichnet, dass** der Tank eine Flüssigkeit enthält, die mindestens einen Wirkstoff aufweist, der aus der Gruppe ausgewählt wird, die folgenden Behandlungswirkstoffe umfasst:
- Methotrexat,
- Adrenalin,
- Sumatriptan,
- Hydrocortison,
- Naloxon,
- Midazolam,
- Apomorphin,
- Ethylnatrexonbromid,
- Phytomenadion,
- Chlorpromazinhydrochlorid,
- Zuclopenthixolacetat,
- Danaparoid-Natrium,
- Enoxaparin-Natrium,
- Estradiolcypionat,
- Medoxyprogesteronacetat,
- Medroparin-Calcium,
- Methylprednisolonacetat,
- Heparinkalzium,
- Terbulin.

## Claims

1. A method for air quenching a glass hollow body (2) elongated along a main axis, including a wall having an external surface (10), and an internal surface (12) formed by a bore extending in height along the main axis, this method using air blast nozzles (22, 28) directed towards the surfaces, **characterized in that** it simultaneously blasts air jets by external nozzles (22) distributed on the external surface (10), and above the bore in the axis by an internal air jet forming in a transverse plane a crown having a hollow (68) in the center.

2. The quenching method according to claim 1, **characterized in that** it blasts air through an axial nozzle (28) opening above the bore.

3. The quenching method according to claim 2, **characterized in that** it blasts air through an axial nozzle (28) comprising a shape which opens forming a crown (64).

4. The quenching method according to any one of the preceding claims, **characterized in that** it blasts air by external nozzles (22) having an axial slot.

5. The quenching method according to claim 4, **characterized in that** all the external nozzles (22) have an axial slot which extends substantially over the entire height of the external surface (10) to be treated.

6. The quenching method according to any one of the preceding claims, **characterized in that** during quenching it rotates the air jet relative to the hollow body (2) about the main axis.

7. A quenching installation of a glass hollow body (2), including nozzles (22, 28) for blasting air on said body, **characterized in that** it includes an axial nozzle (28) having a shape that opens comprising an axial core connected by radiuses (66) to an external contour and means implementing a method according to any one of the preceding claims.

8. An injection device carrying out intradermal, subcutaneous or intramuscular needleless injections of active ingredients contained in a fluid for therapeutic use, **characterized in that** it includes a reservoir containing this fluid, formed by a glass tube constituting a hollow body (2) made by a method according to any one of claims 1 to 6.

9. The injection device according to claim 8, **characterized in that** the reservoir contains a fluid having at least one active ingredient selected from the group comprising the following treatment active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medroxyprogesterone acetate,
- Medroparin calcium,
- Methylprednisolone acetate,
- Heparin calcium,
- Terbuline.
